# EUROPEAN PATENT SPECIFICATION

(11) **EP 3 484 475 B1**
(45) Date of publication and mention of the grant of the patent: **27.09.2023**
(21) Application number: 17743399.2
(22) Date of filing: 16.07.2017
(51) Int. Cl.: A61K 31/455, A61K 45/06, A61P 1/04, A61K 9/00, A61P 9/00, A61P 11/00, A61P 13/12, A61P 17/02, A61P 29/00, A61P 31/00, A61P 9/04, A61P 9/10, A61P 19/02, A61P 21/02, A61P 25/00, A61P 31/06, A61P 31/04, A61P 35/00, A61P 43/00

(54) **1-METHYLNICOTINAMIDE FOR THE TREATMENT OF CARDIOVASCULAR DISEASE**
1-METHYLNICOTINAMID ZUR BEHANDLUNG VON CARDIOVASKULÄREN ERKRANKUNGEN
1-MÉTHYLNICOTINAMIDE DESTINÉ AU TRAITEMENT DES MALADIES CARDIOVASCULAIRES

(30) Priority: 18.07.2016 US 201662363712 P
(43) Date of publication of application: 22.05.2019
(73) Proprietor: Pharmena S.A., 90-530 Lodz (PL)
(72) Inventor: PALKA, Konrad, 94-131 Lodz (PL); GEBICKI, Jerzy, 90-010 Lodz (PL); WIECZORKOWSKA, Marzena, 92-512 Lodz (PL); CEFALI, Eugenio A., Fort Lauderdale FL 33301 (US)
(74) Representative: Patpol Kancelaria Patentowa Sp. z o.o.
(86) International application number: PCT/IB2017/054293
(87) International publication number: WO 2018/015861

(56) References cited:
- WO-A1-2006/024545
- WO-A1-2008/104920
- WO-A1-2014/200373
- WO-A2-2005/067927
- WO-A2-2007/074406
- WO-A2-2007/103540
- WO-A2-2009/047291
- CHLOPICKI S ET AL: "1-Methylnicotinamide (MNA), a primary metabolite of nicotinamide, exerts anti-thrombotic activity mediated by a cyclooxygenase-2/prostacyclin pathway", BRITISH JOURNAL OF PHARMACO, WILEY-BLACKWELL, UK, vol. 152, 1 January 2007 (2007-01-01), pages 230-239, XP007905228, ISSN: 0007-1188, DOI: 10.1038/SJ.BJP.0707383

## Description

This invention relates to the treatment of diseases associated with C-reactive protein.

### BACKGROUND OF THE INVENTION

1-Methylnicotinamide (1-MNA) is a quaternary pyridinium compound. It is a metabolite of nicotinamide. 1-MNA can exist in various pharmaceutically acceptable salt forms, e.g., 1-MNA chloride.

C-reactive protein (CRP), a plasma protein synthesized by the liver, is a sensitive and dynamic systemic marker of inflammation. CRP is a ring-shaped, acute-phase, pentameric protein, the levels of which rise in response to inflammation following interleukin-6 secretion by macrophages and T cells. CRP binds to lysophosphatidylcholine expressed on the surface of dead or dying cells and certain types of bacteria to activate the complement system via the C1Q complex. Thompson, D. et al. "The physiological structure of human C-reactive protein and its complex with phosphocholine" Structure 7 (2): 169-77 (1999). CRP is a member of the pentraxin family of proteins. CRP is a pattern recognition receptor (PRR) of the innate immune system for identifying the presence of antigens. The amount of CRP in blood or serum levels can be determined by blood tests.

CRP levels are elevated in all diseases that have some degree of inflammation. CRP rises within two hours of the onset of inflammation and peaks at 48 hours. Its half-life is about 18 hours. The CRP level depends on its rate of production, which correlates with the severity of the precipitating cause. An elevated CRP level is considered to be a "marker" for inflammatory diseases. Declining or lowering of CRP levels is considered as an indicator of decreased inflammation.

The CRP concentration in the circulation can increase by up to 10,000-fold during acute responses to serious infection or major tissue damage. Typically, blood or serum CRP levels of greater than 10 mg/L indicate the presence of at least one ongoing inflammatory disease. Various studies suggest that chronic internal inflammation can lead to many serious, age-related diseases including heart disease, some forms of cancer and neurodegenerative conditions such as Alzheimer's and Parkinson's disease.

The identification of individuals who are at risk for developing cardiovascular disease but who currently lack symptoms is important in primary prevention. Cardiovascular risk prediction algorithms have relied on core risk factors such as blood pressure, smoking status, hyperlipidemia or dyslipidemia, and the presence or absence of diabetes. Dyslipidemia is a disorder with an abnormal amount of lipids in the blood. Dyslipidemia is characterized by an elevated blood triglyceride level (TG), an elevated blood low-density lipoproteins (LDL), an elevated total blood cholesterol, a low level of blood high-density lipoproteins (HDL), an elevated blood apolipoprotein B (apo B) level, an elevated blood apolipoprotein A1 (apo A1), or a combination thereof.

These core traditional risk factors for heart disease and stroke derive largely from the Framingham Heart Study in the early 1960s. These risk factors and their interactions with age and sex were codified in the 1980s into the Framingham Risk Score, which has been used to evaluate an individual's risk for cardiovascular diseases.

The Framingham Risk Score has limitations as they do not always adequately predict the risk of cardiovascular diseases (e.g., coronary heart disease (CHD)). See Risk factors for atherosclerosis, Highlights, XV International Symposium on Atherosclerosis, p. 2, (June 14-18, 2009). For example, individuals suffer cardiovascular diseases despite being classified as being at low risk for the diseases by the traditional factors (e.g., the level of blood lipids such as TG and/or LDL-cholesterol within the normal range). Most vascular events have been reported to occur among individuals without evidence of very high LDL-cholesterol levels. Ridker P. M. et al., "Should C-Reactive Protein Be Added to Metabolic Syndrome and to Assessment of Global Cardiovascular Risk?" Circulation 109:2818-2825 (2004). And about 20% of cardiovascular events occur in individuals in whom traditional risk factors have not been identified.

Blood or serum CRP level has been reported to be associated with the risk of cardiovascular diseases (e.g., coronary heart disease, ischemic stroke, and vascular mortality). It has been used as a marker independently or together with the traditional factors for cardiovascular risks as well as being a target for reducing the risk for cardiovascular diseases. *See* The Emerging Risk Factors Collaboration "C-reactive Protein Concentration and Risk of Coronary Heart Disease, Stroke, and Mortality: An Individual Participant Meta-analysis," The Lancet 375:132-140 (2010); Musunuru K. et al., "The Use of High-sensitivity Assays for C-reactive Protein in Clinical Practice," Nature Clinical Practice 5(10):621-635 (2008). CRP can be more useful and accurate than the traditional factors in predicting cardiovascular disease risks, especially in the subpopulation of individuals where traditional factors may be inadequate.

For example, the JUPITER (Justification for the Use of Statins in Primary Prevention: an Intervention Trial Evaluating Rosuvastatin) trial has shown that patients with a CRP level of > 2 mg/L but no hyperlipidemia (e.g., LDL-cholesterol level below 130 mg/dL) benefited from statin therapy (e.g., the reduction of cardiovascular events). Ridker P.M. et al., "The JUPITER Trial Results, Controversies, and Implications for Prevention," Circ. Cardiovasc. Qual. Outcomes. 2:279-285 (2009). The JUPITER trial has also shown that the rate of cardiovascular events was reduced with CRP lowering therapy for patients with a CRP level of < 1.0 mg/ml. These results show that CRP can be reduced in a LDL-cholesterol independent manner and that CRP is an independent therapeutic target in the prevention and/or treatment of cardiovascular diseases.

Elevated levels of CRP are also observed in many inflammatory diseases such as rheumatoid arthritis, colon cancer, breast cancer, lung cancer, liver cancer, pancreas cancer, infection, inflammatory bowel disease, lupus erythematosus, pneumococcal pneumonia, rheumatic fever, tuberculosis, renal failure, and amyotrophic lateral sclerosis.

US2006104941 discloses the use of a lipid reducing agent, including nicotinic acid and statin, for treating a nonhypercholesterolemic human with an above-normal blood or serum CRP level to reduce the risk of cardiovascular disorder. However, the use of nicotinic acid has been limited because of dose-dependent adverse effects including dyspepsia, abdominal pain, diarrhea, and particularly flushing. In addition, nicotinic acid is contraindicated in patients with liver disease and should not be used in patients with unstable angina or in the acute phase of myocardial infarct.

WO 2007/074406 discloses 1-MNA chloride, optionally in combination with a statin, for use in the prevention of a lipoprotein abnormality which is a disease or disorder associated with the development and progress of atherosclerosis, angina pectoris or cardiac risk.

WO 2005/067927 discloses the use of quaternary pyridinium salts as vasoprotective agents. In particular, 1-MNA salt is disclosed as being useful in the treatment of cardiovascular diseases such as myocardial infarct and ischaemic stroke. Nevertheless publication does not disclose the use of 1-MNA salts with regard to the human subjects, and in particular with regard to the non-dyslipidaemic human patients.

WO 2006/024545 discloses the use of 1-MNA salts in the treatment of cardiovascular disease such as stroke. In particular, according to WO 2006/024545 1-MNA chloride is used to reduce atherosclerotic plaque formation which, in turn, results in an improvement of vascular function with a consequent reduction in the incidence of heart infarct of stroke.

WO 2014/200373 discloses 1-MNA nitrate for the treatment of cardiovascular diseases such as chronic heart failure and ischaemic episodes.

However, none of the above mentioned publications disclose the use of 1-MNA or its salts in relation to the human subjects, and in particular in relation to the non-dyslipidaemic human subjects.

WO 2007/103450 discloses the use of 1-MNA chloride in animal models. However, the publication is silent about prevention or lowering the risk of a cardiovascular disease.

### BRIEF SUMMARY OF THE INVENTION

The present invention relates to a pharmaceutically-acceptable salt of 1-methylnicotinamide (1-MNA) for use in the prevention of a cardiovascular diseaselowering the serum or blood level of C-reactive protein, in a human subject having a high risk of development of a cardiovascular disease,
wherein a pharmaceutically acceptable salt of 1-methylnicotinamide (1-MNA) is used in a therapeutically effective amount, and,
wherein the cardiovascular disease is selected from the group consisting of coronary heart disease (CHD), peripheral artery disease, carotid artery disease, congestive heart failure, stroke, myocardial infarct, angina or a combination thereof,
and wherein prior to administering the human subject has a blood or serum C-reactive protein (CRP) level of less than 10 mg/L,
and wherein the human subject is a non-dyslipidemic human subject,
and wherein a non-dyslipidemic human subject is a human subject having a normal total blood cholesterol level (≤200 mg/dL), a normal LDL-cholesterol level (≤ 130 mg/dL), a normal TG level (≤ 150 mg/dL), or a normal HDL-cholesterol level (≥60 mg/dL), or a combination thereof,
and wherein the high risk occurs when the human subject has CRP level between 3 mg/L and 10 mg/L.

Preferably, the human subject has a blood C-reactive protein (CRP) in a range selected from the group consisting of: less than 1.0 mg/L, less than 0.5 mg/L, between 0.5 mg/L to less than 1.0 mg/L, between 1.0 mg/L to less than 10.0 mg/L, more preferably between 0.5 mg/L to less than 1.0 mg/L, preferably less than 1.0 mg/L, between 1.0 mg/L to less than 3.0 mg/L, between 1.0 mg/L to 2.0 mg/L, between 1.0 mg/L to 2.7 mg/L, or between 2.0 to less than 3.0 mg/L, 3.0 mg/L to 5.0 mg/L, between 3.0 mg/L to 7.0 mg/L, or between 3.0 mg/to less than 10.0 mg/L. More preferably, human subject has a blood C-reactive protein (CRP) in a range of between 1.0 mg/L to less than 10 mg/L.

Preferably, the therapeutically effective amount of a pharmaceutically acceptable salt of 1-MNA is for oral administration.

According to preferred embodiments of the present invention the pharmaceutically acceptable salt is 1-methylnicotinamide chloride.

A therapeutically effective amount of a pharmaceutically acceptable salt of 1-MNA is preferably in the range of from 1000 to 8000 mg, from 1000 mg to 7000 mg, from 1000 mg to 6000 mg, from 1000 mg to 5000 mg, from 1000 mg to 4000 mg, from 1000 mg to 3000 mg, from 1000 mg to 2000 mg, from 2000 mg to 8000 mg, from 2000 mg to 7000 mg, from 2000 mg to 6000 mg, from 2000 mg to 5000 mg, from 2000 mg to 4000 mg, from 2000 mg to 3000 mg, from 3000 mg to 8000 mg, from 3000 mg to 7000 mg, from 3000 mg to 6000 mg, from 3000 mg to 5000 mg, from 3000 mg to 4000 mg, from 4000 mg to 8000 mg, from 4000 mg to 7000 mg, from 4000 mg to 6000 mg, from 4000 to 5000 mg, from 5000 mg to 8000 mg, from 5000 mg to 7000 mg, from 5000 mg to 6000 mg, from 6000 mg to 8000 mg, from 6000 mg to 7000 mg, from or 7000 mg to 8000 mg, per day, more preferably amounts 1000 mg, 2000 mg, 3000 mg, 4000, 5000 mg, 6000 mg, 7000 mg, or 8000 mg, per day, even more preferably 1000 mg, 3000 mg, or 6000 mg, per day.

In preferred embodiments the therapeutically effective amount of a pharmaceutically acceptable salt 1-MNA is for once a day, twice a day, or three times a day administration

Described herein is also a method for treating a subject to prevent a cardiovascular disease, or to lower or reduce the risk of the cardiovascular disease, comprising administering to the subject a therapeutically effective amount of 1-MNA or a pharmaceutically acceptable salt thereof, wherein the subject has a blood or serum CRP level of less than 10 mg/L. Also described is the use 1-MNA or a pharmaceutically acceptable salt thereof for the preparation of a medicament for preventing cardiovascular disease or lowering the risk of cardiovascular disease in a subject, wherein the subject has a blood or serum CRP level of less than 10 mg/L.

In some aspects, the subject has a blood or serum CRP level of less than 3 mg/L or less than 1 mg/L. In some aspects, the cardiovascular disease is selected from the group consisting of coronary heart disease (CHD), peripheral artery disease, carotid artery disease, congestive heart failure, stroke, myocardial infarct, angina, and a combination thereof. In one aspect, the administration of 1-MNA or a pharmaceutically acceptable salt thereof reduces or lowers the subject's CRP level. In some aspects, the method further comprises administering one or more additional active agents together with the 1-MNA or a pharmaceutically acceptable salt thereof.

In another aspect, described herein is a method of treating a disease associated with elevated or undesired levels of blood or serum CRP levels in a subject, to lower the CRP levels in the subject, or to prevent a disease associated with an increase in CRP levels in the subject, the method comprising administering to the subject a therapeutically effective amount of 1-MNA or a pharmaceutically acceptable salt thereof. Also described is 1-MNA or a pharmaceutically acceptable salt thereof for use in a method of treating a disease associated with elevated or undesired levels of blood or serum CRP levels in a subject, to lower the CRP levels in the subject, or to prevent a disease associated with an increase in CRP levels in the subject. Also described is the use of 1-MNA or a pharmaceutically acceptable salt thereof for the preparation of a medicament for treating a disease associated with elevated or undesired levels of blood or serum CRP levels in a subject, to lower the CRP levels in the subject, or to prevent a disease associated with an increase in CRP levels in the subject. In some aspects, the disease includes rheumatoid arthritis, colon cancer, breast cancer, lung cancer, liver cancer, pancreas cancer, infection, inflammatory bowel disease, lupus erythematosus, pneumococcal pneumonia, rheumatic fever, tuberculosis, renal failure, amyotrophic lateral sclerosis or a combination thereof. In some embodiments, the subject, especially prior to the administration, has a blood or serum CRP level of 10 mg/L or higher. In some embodiments, the treatment reduces or lowers the subject's CRP level.

### DETAILED DESCRIPTION OF THE INVENTION

To facilitate an understanding of the invention disclosed herein, a number of terms and phrases are defined below. The invention is described by the claims.

### Definitions

Unless defined otherwise, all technical and scientific terms used herein have the same meaning as commonly understood by one of ordinary skill in the art to which this disclosure is related. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, provide one of skill with a general dictionary of many of the terms used in this disclosure.

In this specification and the appended claims, the singular forms "a", "an" and "the" include plural referents unless the context clearly dictates otherwise. The terms "a" (or "an"), as well as the terms "one or more," and "at least one" can be used interchangeably herein.

Furthermore, "and/or" where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Numeric ranges are inclusive of the numbers defining the range. Where a range of values is recited, it is to be understood that each intervening integer value, and each fraction thereof, between the recited upper and lower limits of that range is also specifically disclosed, along with each subrange between such values. The upper and lower limits of any range can independently be included in or excluded from the range, and each range where either, neither or both limits are included is also encompassed within the invention. Where a value is explicitly recited, it is to be understood that values that are about the same quantity or amount as the recited value are also disclosed. Where a combination is disclosed, each subcombination of the elements of that combination is also specifically disclosed. Conversely, where different elements or groups of elements are individually disclosed, combinations thereof are also disclosed. Where any element of an invention is disclosed as having a plurality of alternatives, examples of that invention in which each alternative is excluded singly or in any combination with the other alternatives are also hereby disclosed; more than one element of an invention can have such exclusions, and all combinations of elements having such exclusions are hereby disclosed.

The term "about" as used herein refers to that the number comprehended is not limited to the exact number set forth herein, and is intended to refer to ranges substantially within the quoted range while not departing from the scope of the invention. As used herein, "about" will be understood by persons of ordinary skill in the art to account for margin of errors or measurement errors, for example, in the range of up to plus or minus 10% of the particular term.

As used herein the term "C reactive protein" or "CRP" refers to an annular (ring-shaped), pentameric protein found in blood or serum the levels of which rise in response to inflammation. CRP is an acute-phase protein synthesized by the liver that increases following interleukin-6 secretion by macrophages and/or T cells. Pepys M.B. and Hirschfield G.M. "C-reactive protein: a critical update" The Journal of Clinical Investigation 111(12): 1805-12 (2003).

In humans, the gene encoding CRP is on the first chromosome (1q21-q23). Human CRP has 224 amino acids and a monomer molecular mass of 25,106 Da. The human CRP has the GenBank Accession No. CAA39671. A blood or serum CRP level of less than 10 mg/L is indicative of no ongoing inflammation while a blood or serum CRP level of 10 mg/L or higher indicates the presence of inflammation. *See* Thomas, L. Labor und Diagnose p. 1010, TH-Books, Frankfurt (2008).

As used herein the terms "treat," "treating", "treating of", "treatment," or "treatment of" refers to (i) reducing the potential for a disease or disorder (e.g., a cardiovascular disease such as CHD or other diseases disclosed herein), (ii) reducing the occurrence of a disease or disorder, (iii) reducing the severity of a disease or disorder, preferably, to an extent that the subject suffers less or no longer suffers discomfort and/or altered function due to it, (iv) reducing an indication or marker of a disease or disorder such as reducing the blood or serum CRP level, or (v) a combination thereof.

For example, treating can refer to the ability of a therapy when administered to a subject, to prevent a disease or disorder from occurring and/or to cure or to alleviate the disease or disorder's symptoms, signs, or causes. Treating also refers to mitigating or decreasing at least one clinical symptom and/or inhibition or delay in the progression of the condition and/or prevention or delay of the onset of a disease or illness. Thus, the terms "treat," "treating" or "treatment of" (or grammatically equivalent terms) refer to both prophylactic and therapeutic treatment regimes.

The phrase "lowering or reducing the risk of cardiovascular disease" refers to (i) preventing cardiovascular disease or disorder (e.g., CHD), reducing the potential or possibility of cardiovascular disease or disorder, (ii) reducing the occurrence of cardiovascular disease or disorder, or (iii) reducing an indicator or marker of cardiovascular risk, such as reducing the blood or serum CRP level.

As used herein the phrase "diseases associated with C-reactive protein" refers to diseases in which association or relationship or link with blood or serum CRP levels can be established or which. The phrase includes diseases associated with blood or serum CRP levels of less than 10 mg/L as well as diseases associated with blood or serum level of 10 mg/L or higher. For example, the phrase include risk of cardiovascular disease (e.g., CHD), diseases such as rheumatoid arthritis, colon cancer, breast cancer, lung cancer, liver cancer, pancreas cancer, infection, inflammatory bowel disease, lupus erythematosus, pneumococcal pneumonia, rheumatic fever, tuberculosis, renal failure, amyotrophic lateral sclerosis, or a combination thereof.

The terms "subject," "individual" or "patient" as used herein refer to any subject, particularly a mammalian subject, for whom diagnosis, prognosis, preventing or therapy of a disease or disorder (e.g., a cardiovascular disease such as CHD or other diseases disclosed herein) is desired. As used herein, the terms "subject," "individual" or "patient" include any human or nonhuman animal. The term "nonhuman animal" includes all vertebrates, *e.g*., mammals and non-mammals, such as mice, nonhuman primates, sheep, dogs, cats, horses, cows, bears, chickens, amphibians, reptiles, *etc.* In preferred embodiments, a subject is a human.

As used herein, the term "blood CRP level" refers to blood, plasma, or serum CRP level unless otherwise clear from the context. The terms "blood CRP level", "plasma CRP level", and "serum CRP level" are used interchangeably herein unless otherwise clear from the context.

The term "therapeutically effective amount" as used herein refers to an amount of a drug effective to "treat" a disease or disorder in a subject or reduce the risk, potential, possibility or occurrence of a disease or disorder (e.g., a cardiovascular disease such as CHD or other disease disclosed herein). A "therapeutically effective amount" includes an amount of a drug or a therapeutic agent that provides some improvement or benefit to a subject having or at risk of having a disease or disorder (e.g., a cardiovascular disease such as or other disease disclosed herein). Thus, a "therapeutically effective" amount is an amount that reduces the risk, potential, possibility or occurrence of a disease or disorder, or provides some alleviation, mitigation, and/or reduction of at least one indicator (e.g., blood or serum CRP level), and/or decrease in at least one clinical symptom of a disease or disorder (e.g., a cardiovascular disease such as CHD or other disease disclosed herein).

The term "administration" or "administering" of a drug or a medication, as used herein, includes delivering, applying, or giving the therapy or drug to a subject including self-administering by the subject.

As used herein, the term "1-methylnicotinamide," also known as 1-MNA, is a quaternary pyridinium compound the cation of which has the structural formula of

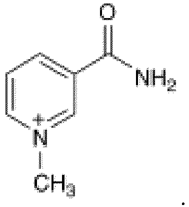

The term "pharmaceutically acceptable salt" refers to a salt of an acidic or basic group of a base compound that is generally safe, non-toxic, neither biologically nor otherwise undesirable, and useful for either or both veterinary use and/or human pharmaceutical use. The disclosure herein discloses 1-MNA, which is capable of forming a wide variety of salts with anions of various inorganic and organic acids. By "pharmaceutically acceptable salt of 1-MNA" it is understood a compound of the formula 1-MNAX⁻, wherein X⁻ is a pharmaceutically acceptable anion.

### Methods of Reducing or Lowering Risk of Cardiovascular Diseases in Patients with a Blood or Serum CRP Level of 10 mg/L or Less

The disclosure herein is based on the finding that 1-MNA or a pharmaceutically acceptable salt thereof reduces blood or serum CRP levels in a subject (e.g., a human). Advantageously, the administration of 1-MNA or a pharmaceutically acceptable salt thereof does not have many of the side-effects that nicotinic acids has (e.g., flushing, hepatotoxicity, gout). In one embodiment, the disclosure herein provides a method of preventing cardiovascular disease or lowering or reducing the risk of cardiovascular disease in a subject who has a blood or serum CRP level of less than 10 mg/L, the method comprising administering to the subject a therapeutically effective amount of 1-MNA or a pharmaceutically acceptable salt thereof. In one embodiment, the cardiovascular disease includes CHD, peripheral artery disease, carotid artery disease, congestive heart failure, stroke, myocardial infarct, angina, or a combination thereof. In some embodiments, the method reduces the risk of CHD. In other embodiments, the method reduces the risk of stroke, myocardial infarct, or a combination thereof in the subject. In some embodiments, the subject is a human. Reducing or lowering blood or serum CRP level lowers the risk of cardiovascular disease.

Without intending to be bound by any theory, it is believed that CRP may play a role in the pathogenesis of cardiovascular disease via a direct pro-inflammatory effect and may initiate atherosclerosis by various mechanisms. Elevated blood or serum CRP levels are indicative of inflammation, which may play a role in the pathogenesis of cardiovascular diseases such as acute atherothrombotic events and atherosclerosis. CRP may be present in vascular cells without any clinical signs of atherosclerotic narrowing of a vessel, such as coronary vessels, shown for example by angiography or ultrasonography. Individuals with low CRP (e.g., CRP<1.0 mg/L) may develop atherosclerosis at a slower rate than individuals with a higher CRP level (e.g., CRP>3.0 mg/L).

In evaluating cardiovascular risks, blood or serum CRP range from zero to 3.0 mg/L and up to about 10.0 mg/L is important. A CRP level of less than 1.0 mg/L is considered low risk for cardiovascular diseases; a CRP level of between 1.0 m/L and less than 3.0 mg/L is considered average risk for cardiovascular diseases; and a CRP level of between 3.0 mg/L and less than 10.0 mg/L is considered high risk for cardiovascular disease.

In some embodiments, the subject has a CRP level of less than 1.0 mg/L. In some embodiments, the subject has a CRP level of less than 0.5 mg/L. In some embodiments, the subject has a CRP level of between 0.5 mg/L to less than 1.0 mg/L.

In some embodiments, the subject has a CRP level of between 1.0 mg/L to less than 10.0 mg/L. In some embodiments, the subject has a CRP level of between 1.0 mg/L to less than 3.0 mg/L, between 1.0 mg/L to 2.0 mg/L, between 1.0 mg/L to 2.7 mg/L, or between 2.0 to less than 3.0 mg/L. In some embodiments, the subject has a CRP level of between 3.0 mg/L to 5.0 mg/L, between 3.0 mg/L to 7.0 mg/L, or between 3.0 mg/L to less than 10.0 mg/L.

In some embodiments, the subject is a non-dyslipidemic subject (e.g., a human). There are general guidelines for dyslipidemia indicators, i.e. blood HDL-cholesterol, LDL-cholesterol, TG, apo A1, apo B, and total cholesterol levels in humans. For example, the target LDL-cholesterol level is less than 130 mg/dL (3.35 mmol/L) or less than 160 mg/dL (4.15 mmol/L). The target HDL-cholesterol level is 60 mg/dL or greater, the target TG level is 150 mg/dL or less, and the target total cholesterol level is 200 mg/dL (5.15 mmol/L) or less. The target apo B level is <80 mg/dL. See e.g., American Association of Clinical Endocrinologists' Guidelines for Management of Dyslipidemia and Prevention of Atherosclerosis (AACE Guidelines), accessible at https://www.aace.com/files/lipid-guidelines.pdf. Blood TG, LDL-cholesterol, HDL-cholesterol, apo B, and apo A1 levels can be determined by methods well known in the art. Total blood cholesterol level can be calculated by methods known in the art, e.g., using the following equation: HDL-cholesterol + LDL-cholesterol + 20 percent of TG level.

In some embodiments, the subject (e.g., a human) being treated is a non-dyslipidemic subject, e.g., has a normal total blood cholesterol level (e.g., ≤200 mg/dL), a normal LDL-cholesterol level (e.g., ≤130 mg/dL), a normal TG level (e.g., ≤150 mg/dL), or a normal HDL-cholesterol level (e.g., ≥60 mg/dL), or a combination thereof.

In some embodiments, the method reduces or lowers the blood or serum CRP level in the subject as compared to that before the treatment. In some embodiments, the treatment does not cause flushing in the subject. In some embodiments, the subject is a human.

In some embodiments, a pharmaceutically acceptable salt of 1-MNA is administered to the subject. Non-limiting pharmaceutically acceptable salts of 1-MNA include sulfate, chloride, bromide, iodide, nitrate, bisulfate, phosphate, acid phosphate, methanesulfonate, ethanesulfonate, benzenesulfonate, p-toluenesulfonate, amino acid salts, salts of mono-, di- and tricarboxylic acids, e.g., acetate, benzoate, salicylate, hydroxyacetate, lactate, maleate, malonate, malate, tartrate, bitartrate, isonicotinate, oleate, tannate, pantothenate, bitartrate, ascorbate, succinate, gentisinate, fumarate, gluconate, glucuronate, saccharate, formate, benzoate, glutamate, pamoate (i.e., 1,1'-methylene-bis-(2-hydroxy-3-naphthoate)), oxalate and citrate salts. In some embodiments, a pharmaceutically acceptable salt includes chloride, benzoate, salicylate, acetate, citrate, and lactate salt of 1-MNA. In some embodiments, a salt of 1-MNA is 1-MNA chloride, also known as TRIA-662. 1-MNA chloride is commercially available (e.g., Sigma, Cayman Chemical).

Pharmaceutically acceptable 1-MNA salts can be prepared from nicotinamide by methods known to persons skilled in the art. Salts with halogen anion can be prepared from nicotinamide by direct methylation with methyl halogenide as known in the art, e.g., as described in AT131118, GB348345, US3614408, and US4115390. Salts with a non-halogen anion can be prepared by substitution of a halogen anion to another anion, for example by treatment with a salt of the another anion, such as for example sodium or silver salt of the another anion. As an illustration, lactate and acetate can be prepared by the treatment of a halogenide, preferably chloride, with silver lactate or acetate, respectively. Salicylate can be prepared by the treatment of a halogenide, preferably chloride, with sodium salicylate.

### Methods of Treating Diseases in a Subject with a Blood or Serum CRP level of Higher Than 10 mg/L

In one aspect, the present publication describes a method of treating a disease associated with elevated or undesired levels of blood or serum CRP levels (e.g., a CRP level of higher than 10 mg/L) in a subject, to lower the CRP levels in the subject, or to prevent a disease associated with increase in CRP levels in the subject. The method comprises administering to the subject a therapeutically effective amount of 1-MNA or a pharmaceutically acceptable salt thereof. For example, CRP levels greater than 100.0 mg/L are associated with major trauma and severe infection (sepsis). CRP levels between 10.0 mg/L and 40.0 mg/L are associated with mild inflammation and viral infections. CRP levels between 40.0 mg/L and 200.0 mg/L are associated with active inflammation and bacterial infection.

It is described that sometimes the disease includes rheumatoid arthritis, colon cancer, breast cancer, lung cancer, liver cancer, pancreas cancer, infection, inflammatory bowel disease, lupus erythematosus, pneumococcal pneumonia, rheumatic fever, tuberculosis, renal failure, amyotrophic lateral sclerosis or a combination thereof. In some embodiments, the disease is rheumatoid arthritis, infection, inflammatory bowel disease, lupus erythematosus, pneumococcal pneumonia, rheumatic fever, tuberculosis, renal failure, amyotrophic lateral sclerosis, or a combination thereof. In some embodiments, the disease is rheumatoid arthritis, inflammatory bowel disease, lupus erythematosus, pneumococcal pneumonia, rheumatic fever, renal failure, or a combination thereof. In some embodiments, the disease is rheumatoid arthritis, inflammatory bowel disease, lupus erythematosus, or a combination thereof. In some embodiments, the disease is rheumatoid arthritis, inflammatory bowel disease, amyotrophic lateral sclerosis, or a combination thereof. In some embodiments, the disease is rheumatoid arthritis. In some embodiments, the disease is amyotrophic lateral sclerosis.

As described, the subject has a blood or serum CRP level of between 10 mg/L and about 100 mg/L, between 10 mg/L and about 90 mg/L, between 10 mg/L and 80 mg/L, between 10 mg/mL and 70 mg/mL, between 10 mg/mL and 60 mg/L, between 10 mg/mL and 50 mg/L, between 10 mg/mL and 40 mg/L, between 10 mg/mL and 30 mg/L, between 10 mg/mL and 20 mg/L, between 20 mg/mL and 100 mg/L, between 20 mg/mL and 80 mg/L, between 20 mg/mL and 60 mg/L, between 20 mg/mL and 40 mg/L, between 30 mg/mL and 100 mg/L, between 20 mg/mL and 80 mg/L, between 30 mg/mL and 60 mg/L, between 30 mg/mL and 40 mg/L, between 40 mg/mL and 100 mg/L, between 40 mg/mL and 80 mg/L, between 40 mg/mL and 60 mg/L, between 50 mg/mL and 100 mg/L, between 50 mg/mL and 80 mg/L, between 50 mg/mL and 60 mg/L, between 60 mg/mL and 100 mg/L, between 60 mg/mL and 80 mg/L, between 70 mg/mL and 100 mg/L, between 70 mg/mL and 80 mg/L, between 80 mg/mL and 100 mg/L, between 80 mg/mL and 60 mg/L, or between 90 mg/mL and 100 mg/L.

As described, the treatment reduces or lowers the blood or serum CRP level in the subject as compared to that before the treatment. In some embodiments, the treatment does not cause flushing in the subject. In some embodiments, the subject is a human.

### Dose of 1-MNA or a Pharmaceutically Acceptable Salt Thereof and Route of Administration

In the solutions described and disclosed herein, the therapeutically effective amount of 1-MNA or a pharmaceutically acceptable salt thereof includes an amount effective to reduce the subject's blood or serum CRP level as compared to that before the treatment. In some embodiments, the therapeutically effective amount of 1-MNA or a pharmaceutically acceptable salt thereof for preventing cardiovascular disease or lowering or reducing the risk of cardiovascular disease includes an amount effective to lower or reduce the subject's blood or serum CRP level as compared to that before the treatment. The therapeutic effective amount of the 1-MNA or a pharmaceutically acceptable salt thereof for the treatment may depend on factors including the blood or serum CRP level of the subject before the treatment, the blood lipids level (e.g., LDL-cholesterol, TG, HDL-cholesterol, or total cholesterol level) in the subject, the presence or absence of other conditions (e.g., presence or absence of diabetes), age and gender of the subject and can be adjusted by a person of ordinary skill in the art (e.g., a doctor).

In some embodiments, the therapeutically effective amount of 1-MNA or a pharmaceutically acceptable salt thereof for treating diseases associated with an increased or elevated blood or serum CRP level (e.g., a CRP level greater than 10 mg/L) includes an amount effective to reduce the subject's blood or serum CRP level as compared to that before the treatment. Such diseases include rheumatoid arthritis, certain cancers, infection, inflammatory bowel disease, lupus erythematosus, pneumococcal pneumonia, rheumatic fever, tuberculosis, renal failure, amyotrophic lateral sclerosis, or a combination thereof. The therapeutic effective amount of the 1-MNA or a pharmaceutically acceptable salt thereof for the treatment may depend on factors including the type disease, the degree of the condition (e.g., severity of the disease), age of the subject and whether the subject has two or more diseases or conditions (e.g., rheumatoid arthritis and infection) and can be adjusted by a person of ordinary skill in the art (e.g., a doctor).

In some embodiments, the therapeutically effective amount of 1-MNA or a pharmaceutically acceptable salt thereof is from 1000 to about 8000 mg, from about 1000 mg to about 7000 mg, from about 1000 mg to about 6000 mg, from about 1000 mg to about 5000 mg, from about 1000 mg to about 4000 mg, from about 1000 mg to about 3000 mg, from about 1000 mg to about 2000 mg, from about 2000 mg to about 8000 mg, from about 2000 mg to about 7000 mg, from about 2000 mg to about 6000 mg, from about 2000 mg to about 5000 mg, from about 2000 mg to about 4000 mg, from about 2000 mg to about 3000 mg, from about 3000 mg to about 8000 mg, from about 3000 mg to about 7000 mg, from about 3000 mg to about 6000 mg, from about 3000 mg to about 5000 mg, from about 3000 mg to about 4000 mg, from about 4000 mg to about 8000 mg, from about 4000 mg to about 7000 mg, from about 4000 mg to about 6000 mg, from about 4000 to about 5000 mg, from about 5000 mg to about 8000 mg, from about 5000 mg to about 7000 mg, from about 5000 mg to about 6000 mg, from about 6000 mg to about 8000 mg, from about 6000 mg to about 7000 mg, from about or 7000 mg to about 8000 mg, per day.

In some embodiments, the therapeutically effective 1-MNA or a pharmaceutically acceptable salt thereof is about 1000 mg, about 2000 mg, about 3000 mg, about 4000, about 5000 mg, about 6000 mg, about 7000 mg, or about 8000 mg, per day. In some embodiments, the therapeutically effective amount of 1-MNA or a pharmaceutically acceptable salt thereof is about 1000 mg, about 3000 mg, or about 6000 mg, per day.

In some embodiments, the therapeutically effective amount of 1-MNA or a pharmaceutically acceptable salt thereof is administered once a day, twice a day, or three times a day. In some embodiments, the therapeutically effective amount of 1-MNA or a pharmaceutically acceptable salt thereof is administered more than three times a day. A therapeutically effective amount can be administered in one dose or divided into multiple doses, as long as the dose is sufficiently high that the subject benefits from the dose or treatment.

The therapeutically effective amount of 1-MNA or a pharmaceutically acceptable salt thereof can be administered by the subject or by one other than the subject systemically (e.g., orally, transmucosally, or via injection) or locally (e.g., topically or via suppository) via various routes known in the art. Some exemplary routes of administration include oral, topical (e.g., transdermal), transmucosal (e.g., buccal and sublingual), injectable (e.g., intravenous, intramuscular, and subcutaneous), and inhalation (e.g., aerosol). In some embodiments, the therapeutically effective amount of 1-MNA or a pharmaceutically acceptable salt thereof is administered orally. In some embodiments, the therapeutically effective amount of 1-MNA or a pharmaceutically acceptable salt thereof is administered transmucosally. In some embodiments, the therapeutically effective amount of 1-MNA or a pharmaceutically acceptable salt thereof is administered via injection. In some embodiments, the treatment does not cause flushing in the subject. In some embodiments, the therapeutically effective amount of the 1-MNA or a pharmaceutically acceptable salt thereof is administered once a day, twice a day, or three times a day, preferably in an oral dosage form.

The 1-MNA or a pharmaceutically acceptable salt thereof is administered as long as the subject benefits from the administration (e.g., blood or serum CRP is reduced or maintained at a reduced level as compared to that before the treatment). In some embodiments, the 1-MNA or a pharmaceutically acceptable salt thereof is administered for at least one month, at least three months, at least six months, at least one year, at least three years, or at least five years.

### Combination Therapy

When used for reducing or lowering risk of cardiovascular disease, the 1-MNA or a pharmaceutically acceptable salt thereof can be administered together with one or more additional active agents either at the same time or separately. The one or more additional active agents include aspirin and/or drugs from various lipid-lowering drug classes, including (1) HMG-CoA reductase inhibitors (e.g., statins such as lovastatin, pravastatin, fluvastatin, simvastatin, atorvastatin, rosuvastatin, and pitavastatin), (2) fibric acid derivatives (e.g., gemfibrozil, fenofibrate, and fenofibric acid), (3) bile acid sequestrants (e.g., cholestyramine, colestipol, colesevelam, and hydrochloride), (4) cholesterol absorption inhibitors (e.g., ezetimibe), or a combination thereof (e.g., ezetimibe/simvastatin). In some embodiments, the 1-MNA or a pharmaceutically acceptable salt thereof is used together with aspirin, a statin or a combination thereof. In some embodiments, the 1-MNA or a pharmaceutically acceptable salt thereof is used in combination with aspirin. In other embodiments, the 1-MNA or a pharmaceutically acceptable salt thereof is used in combination with a statin such as lovastatin, pravastatin, fluvastatin, simvastatin, atorvastatin, rosuvastatin, or pitavastatin. The amount of the one or more additional active agents is known in the art, e.g., see the AACE Guidelines. In some embodiments, the method reduces or lowers the blood or serum CRP level in the subject as compared to that before the treatment. In some embodiments, the treatment does not cause flushing in the subject. In some embodiments, the subject is a human.

In treating diseases associated with increased, elevated or undesired blood or serum CRP levels (e.g., a CRP level of higher than 10 mg/L) in a subject, administering of the pharmaceutically-acceptable salt of 1-methylnicotinamide (1-MNA) can further comprise administering one or more additional active agent to the subject. Such diseases include rheumatoid arthritis, colon cancer, breast cancer, lung cancer, liver cancer, pancreas cancer, infection, inflammatory bowel disease, lupus erythematosus, pneumococcal pneumonia, rheumatic fever, tuberculosis, renal failure, amyotrophic lateral sclerosis, or a combination thereof. In one embodiment, the disease is rheumatoid arthritis and the method further comprising administering one or more active agent that treats this disease. In one embodiment, the disease is infection (e.g., pneumococcal pneumonia, rheumatic fever, tuberculosis) and the method further comprising administering one or more active agent that treats infection. In one embodiment, the disease is inflammatory bowel disease and the method further comprising administering one or more active agent that treats this disease. In one embodiment, the disease is lupus erythematosus and the method further comprising administering one or more active agent that treats lupus erythematosus. In one embodiment, the disease is colon cancer and the method further comprising administering one or more active agent that treats colon cancer. In one embodiment, the disease is breast cancer and the method further comprising administering one or more active agent that treats breast cancer. In one embodiment, the disease is lung cancer and the method further comprising administering one or more active agent that treats lung cancer. In one embodiment, the disease is liver cancer and the method further comprising administering one or more active agent that treats liver cancer. In one embodiment, the disease is pancreas cancer and the method further comprising administering one or more active agent that treats pancreas cancer. In one embodiment, the disease is renal failure and the method further comprising administering one or more active agent that treats the disease or using a procedure for treating renal failure (e.g., hemodialysis). In one embodiment, the disease is amyotrophic lateral sclerosis and the method further comprising administering one or more active agent that treats the disease.

Exemplary active agents useful for use in case of rheumatoid arthritis include steroid, nonsteroidal anti-inflammatory drug (NSAID), methotrexate, hydroxycholorquine, sulfasalazine, leflunomide, cyclophosphamide, azathioprine, tofacitinib, abatacept (Orencia^{®}), adalimumab (Humira^{®}), anakinra (Kineret^{®}), certolizumab (Cimzia^{®}), etanercept (Enbrel^{®}), golimumab (Simponi^{®}), infliximab (Remicade^{®}), rituximab (Rituxan^{®}), tocilizumab (Actemra^{®}), tofacitinib (Xeljanz^{®}) or a combination thereof.

Exemplary active agents useful for use in case of infection (e.g., pneumococcal pneumonia, rheumatic fever, or tuberculosis) include antibacterial agents such as penicillins, cephalosporins, vancomycin (Vancocin^{®}), polymixin, gramicidin, tetracyclines, macrolides, chloramphenicol, clindamycin, spectinomycin, sulfonamides, ciprofloxacin (Cipro^{®}), ofloxacin (Floxin^{®}), isoniazid (INH^{®}), rifampicin, pyrazinamide, ethambutol (Myambutol^{®}), streptomycin, or a combination thereof.

Exemplary active agents useful for use in case of inflammatory bowel disease include mesalamine (Lialda^{®}), balsalazide (Colazal^{®}), olsalazine (Dipentum^{®}), prednisone, hydrocortisone, Azathioprine, (Azasan^{®}) mercaptopurine (Purinethol^{®}), cyclosporine, infliximab (Remicade^{®}), adalimumab (Humira^{®}), golimumab (Simponi^{®}), methotrexate (Rheumatrex), natalizumab (Tysabri^{®}), vedolizumab (Entyvio^{®}), ustekinumab (Stelara^{®}), or a combination thereof.

Exemplary active agents useful for use in case of lupus erythematosus include prednisone, cortisone, hydrocortisone, NSAID (indomethacin (Indocin^{®}), nabumetone (Relafen^{®}), celecoxib (Celebrex^{®})), chloroquine (Aralen^{®}) and hydroxychloroquine (Plaquenil^{®}), azathioprine (Imuran^{®}), methotrexate (Rheumatrex^{®}), cyclophosphamide (Cytoxan^{®}), or a combination thereof.

Exemplary active agents useful for use in case of colon cancer include 5-fluorouracil (5-FU), capecitabine (Xeloda^{®}), irinotecan (Camptosa^{®}r), oxaliplatin (Eloxatin^{®}), trifluridine, tipiracil (Lonsurf^{®}), or a combination thereof.

Exemplary active agents useful for use in case of breast cancer include anastrozole, bevacizumab, capecitabine, carboplatin, denosumab, docetaxel, doxorubicin, eribulin, exemestane, fluorouracil, fulvestrant, gemcitabine, ixabepilone, lapatinib, letrozole, methotrexate, paclitaxel, trastuzumab, tamoxifen, or a combination thereof.

Exemplary active agents useful for use in case of lung cancer include bevacizumab, carboplatin, cisplatin, crizotinib, docetaxel, doxorubicin, erlotinib, etoposide, gemcitabine, paclitaxel, pemetrexed, vinorelbine, or a combination thereof.

Exemplary active agents useful for use in case of liver cancer include sorafenib tosylate (Nexavar^{®}).

Exemplary active agents useful for use in case of pancreas cancer include Paclitaxel (Abraxane), everolimus (Afinitor ^{®}), erlotinib hydrochloride(Tarceva^{®}), 5-FU (fluorouracil injection), gemcitabine hydrochloride (Gemzar^{®}), irinotecan hydrochloride (Onivyde^{®}), mitomycin C (Mitozytrex^{®}), sunitinib malate (Sutent^{®}), or a combination thereof.

Exemplary therapies useful for use in case of renal failure include hemodialysis.

In some embodiments, the 1-MNA or a pharmaceutically acceptable salt thereof can be administered in combination with one or more additional CRP-lowering drugs in treating diseases associated with CRP such as rheumatoid arthritis, or preventing/reducing or lowering the risk of cardiovascular disease. Exemplary additional CRP-lowering drugs include (1) cyclooxygenase-2 (COX-2) inhibitors (e.g., rofecoxib and celecoxib), (2) antiplatelet agents (e.g., clopidogrel), (3) antidiabetic agents (e.g., thiazolidinedione derivatives such as rosiglitazone, rosiglitazone and pioglitazone), (4) antiestrogen (e.g., tamoxifen), (5) β-adrenoreceptor antagonists (e.g., carvedilol and propranolol), (6) antioxidants (e.g., vitamins E and C, RRR-α-tocopherol acetate), (7) angiotensin converting enzyme (ACE) inhibitors (e.g., ramipril, quinapril, fosinopril, iisinopril, and captopril), (8) angiotensin receptor blockers (e.g., valsartan, losartan, candesartan, irbesartan, and telmisartan), (9) calcium channel antagonists (e.g., verapamil, dihydropyridines, amlodipine, and valsartan), and (10) diuretics (e.g., hydrochlorothiazide).

The one or more additional active agents can be administered together (e.g., at the same time) with the 1-MNA or a pharmaceutically acceptable salt thereof or separately (e.g., before or after), by the subject or one other than the subject. In some embodiments, the one or more additional active agents can be administered systemically or locally via various routes known in the art. Exemplary routes of administration include oral, topical, transmucosal, injectable, and inhalation. In some embodiments the one or more additional active agents are in the same dosage form as the 1-MNA or a pharmaceutically acceptable salt thereof, e.g., an oral dosage form. In some embodiments, the 1-MNA or a pharmaceutically acceptable salt thereof is formulated in the same dosage form with the one or more additional active agents such as aspirin and/or a statin, in certain embodiments, the dosage form is a solid oral dosage form (e.g., tablets or capsules). Dose and dosing regiments of the one or more additional active agents are known in the art, e.g., available from the product label of the drug and/or determined by a medical professional (e.g., a doctor).

### Formulations Comprising 1-MNA or a Pharmaceutically Acceptable Salt Thereof

1-MNA or a pharmaceutically acceptable salt thereof can be formulated in various pharmaceutically compositions for administration. Exemplary pharmaceutical compositions include solutions, suspensions, emulsions, tablets, pills, pellets, powders, multi-particulates, capsules, capsules containing liquids, capsules containing powders, capsules containing multi-particulates, lozenges, sustained-release formulations, suppositories, transdermal patches, transmucosal films, sublingual tablets or films, aerosols, sprays, or any other form suitable for use. Various pharmaceutical compositions and methods for making the compositions are known in art, e.g., as described in described in *Remington's Pharmaceutical Sciences* 1447-1676 (Alfonso R. Gennaro ed., 19th ed. 1995).

When the pharmaceutically effective amount of 1-MNA or a pharmaceutically acceptable salt thereof is administered orally, e.g., comprised in a pharmaceutically acceptable oral dosage form, such oral dosage forms can also comprise a suitable amount of one or more pharmaceutically acceptable excipients, including a diluent, suspending agent, solubilizer, binder, disintegrant, preservative, coloring agent, lubricant. The pharmaceutical excipients can be a liquid, such as water or oil, including those of petroleum, animal, vegetable, or synthetic origin, such as peanut oil, soybean oil, mineral oil, sesame oil. The pharmaceutical excipient can be saline, gum acacia, gelatin, starch paste, talc, keratin, colloidal silica, urea. In addition, auxiliary, stabilizing, thickening, lubricating, and coloring agents can be used. In one embodiment, the pharmaceutically acceptable excipient is sterile when administered to a human subject. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol. The pharmaceutical compositions, if desired, can also contain minor amounts of wetting or emulsifying agents, or pH buffering agents. Examples of pharmaceutically acceptable carriers and excipients that can be used to formulate oral dosage forms are known in the art, e.g., described in the *Handbook of Pharmaceutical Excipients,* American Pharmaceutical Association (1986).

In certain embodiments, 1-MNA or a pharmaceutically acceptable salt thereof is formulated for oral administration in the form of tablets, capsules, gelcaps, caplets, lozenges, aqueous or oily solutions, suspensions, granules, powders, emulsions, syrups, or elixirs, for example. The tablets can be compressed, enteric-coated, sugar-coated, film-coated, multiply compressed or multiply layered.

Oral dosage forms comprising 1-MNA or a pharmaceutically acceptable salt thereof of the present disclosure can contain one or more additional components such as, for example, sweetening agents such as fructose, aspartame or saccharin; flavoring agents such as peppermint, oil of wintergreen, or cherry; coloring agents; preserving agents, and stabilizers, to provide stable, pharmaceutically palatable dosage forms. Techniques and compositions for making solid oral dosage forms are known in the art, e.g., described in Pharmaceutical Dosage Forms: Tablets (Lieberman, Lachman and Schwartz, eds., 2nd ed.) published by Marcel Dekker, Inc. Techniques and compositions for making tablets (compressed and molded), capsules (hard and soft gelatin) and pills are also described in Remington's Pharmaceutical Sciences 1553-1593 (Arthur Osol, ed., 16th ed., Mack Publishing, Easton, PA 1980). Liquid oral dosage forms include aqueous and nonaqueous solutions, emulsions, suspensions, and solutions and/or suspensions reconstituted from non-effervescent granules, optionally containing one or more suitable solvents, preservatives, emulsifying agents, suspending agents, diluents, sweeteners, coloring agents, flavoring agents. Techniques and compositions for making liquid oral dosage forms are known in the art, e.g., described in Pharmaceutical Dosage Forms: Disperse Systems, (Lieberman, Rieger and Banker, eds.) published by Marcel Dekker, Inc.

In certain embodiments, the 1-MNA or a pharmaceutically acceptable salt thereof is delivered in an immediate release form. In other embodiments, 1-MNA or a pharmaceutically acceptable salt thereof of the present disclosure is delivered in a controlled-release system or sustained-release system. Controlled- or sustained-release pharmaceutical compositions can improve drug therapy over the results achieved by their non-controlled or non-sustained-release counterparts. Advantages of controlled- or sustained-release compositions include extended activity of the drug, reduced dosage frequency, and increased compliance. In addition, controlled- or sustained-release compositions can favorably affect the time of onset of action or other characteristics, such as blood levels of the drug compound, and can thus reduce the occurrence of adverse side effects.

Controlled- or sustained-release compositions can initially immediately release an amount of the 1-MNA or a pharmaceutically acceptable salt thereof of the present disclosure that promptly produces the desired therapeutic or prophylactic effect, and gradually and continually release the remaining amounts to maintain a level of therapeutic or prophylactic effect over an extended period of time. Controlled- or sustained-release of an active ingredient can be stimulated by various conditions, including but not limited to, changes in pH, changes in temperature, concentration or availability of enzymes, concentration or availability of water, or other physiological conditions or compounds. Methods for making controlled- or sustained-release oral dosage forms are known in the art, e.g., as described Remington's Pharmaceutical Sciences 1553-1593 (Arthur Osol, ed., 16th ed., Mack Publishing, Easton, PA 1980). In some embodiments, the controlled- or sustained-release can be prepared by methods described in e.g., U.S. Patent No. 5,007,790. Such controlled- or sustained-release oral dosage forms comprise a plurality of particles of a dispersion of a drug in a hydrophilic, water-swellable, crosslinked polymer that maintains its physical integrity over the dosing lifetime but thereafter rapidly dissolves. Once ingested, the particles swell to promote gastric retention and permit the gastric fluid to penetrate the particles, thereby dissolve and release the drug.

Controlled-release and sustained-release means for use according to the present disclosure may be selected from those known in the art. Examples include those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566. Such dosage forms can be used to provide controlled- or sustained-release of one or more active ingredients using, for example, hydroxypropylmethyl cellulose, other polymer matrices, gels, permeable membranes, osmotic systems, multilayer coatings, microparticles, multiparticulates, liposomes, microspheres, or a combination thereof to provide the desired release profile in varying proportions. Suitable controlled- or sustained-release formulations known in the art, including those described herein, can be readily selected for use with the active ingredients of the invention in view of this disclosure. Other controlled- or sustained-release systems that are discussed in the review by Langer, Science 249:1527-1533 (1990) can be selected for use according to the present disclosure.

When 1-MNA or a pharmaceutically acceptable salt thereof of the present disclosure are in a tablet or a pill form, the tablet or pill can be coated to delay disintegration and absorption in the gastrointestinal tract, thereby providing a sustained action over an extended period of time. Selectively permeable membranes surrounding an osmotically active driving compound are also suitable for orally administered compositions. In these latter platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time-delay material such as glycerol monostearate or glycerol stearate can also be used. Oral compositions can include standard excipients such as mannitol, lactose, starch, magnesium stearate, sodium saccharin, cellulose, and magnesium carbonate. In one embodiment, the excipients are of pharmaceutical grade.

Controlled- or sustained-release oral pharmaceutical compositions comprising the 1-MNA or a pharmaceutically acceptable salt thereof include single unit dosage forms such as, tablets, capsules, gelcaps, and caplets. In some embodiments, the oral dosage forms are tablets or capsules.

The pharmaceutical compositions (e.g., oral dosage forms) comprising 1-MNA or a pharmaceutically acceptable salt thereof can be administered once a day, twice a day, three times a day, or more than three times a day. In some embodiments, the pharmaceutical compositions are administered once a day or twice a day. Typically, immediate-release dosage forms are administered more frequently than controlled- or sustained-release dosage forms.

The pharmaceutical compositions can comprise various amount of 1-MNA or a pharmaceutically acceptable salt thereof. In some embodiments, the amount of 1-MNA or a pharmaceutically acceptable salt thereof in a pharmaceutical composition is from 500 mg to 2500 mg, from 500 mg to 2000 mg, from 500 mg to 1500 mg, from 500 mg to 1000 mg, from 600 mg to 2500 mg, from 600 to 2000 mg, from 600 mg to 1500, from 600 mg to 1000 mg, from 700 mg to 2500 mg, from 700 mg to 2000, from 700 mg to 1500, from 700 mg to 1000 mg, from 800 to 2500, from 800 mg to 2000 mg, from 800 to 1500, from 900 mg to 2500, from 900 mg to 2000 mg, from 900 mg to 1500 mg, from 1000 mg to 2500 mg, from 1000 mg to 2000, or from 1000 mg to 1500 mg. In some embodiments, the amount of 1-MNA or a pharmaceutically acceptable salt thereof in a pharmaceutical composition is 500, 600, 700, 800, 900, 1000, 1200, 1300, 1400, 1500, 1600, 1700, 1800, 1900, 2000, 2100, 2200, 2300, 2400, or 2500 mg.

In some embodiments, the pharmaceutical composition comprising 1-MNA or a pharmaceutically acceptable salt thereof disclosed herein further comprises one or more additional agents such as those disclosed above. In some embodiments, the pharmaceutical composition comprising 1-MNA or a pharmaceutically acceptable salt thereof and aspirin and/or drugs from various lipid-lowering drug classes, including (1) HMG-CoA reductase inhibitors (e.g., statins such as lovastatin, pravastatin, fluvastatin, simvastatin, atorvastatin, rosuvastatin, and pitavastatin), (2) fibric acid derivatives (e.g., gemfibrozil, fenofibrate, and fenofibric acid), (3) bile acid sequestrants (e.g., cholestyramine, colestipol, colesevelam, and hydrochloride), (4) cholesterol absorption inhibitors (e.g., ezetimibe), or a combination thereof (e.g., ezetimibe/simvastatin).

In some embodiments, the solid oral composition comprises 1-MNA or a pharmaceutically acceptable salt thereof and aspirin. In some embodiments, the solid oral composition comprises 1-MNA or a pharmaceutically acceptable salt thereof and a statin such as lovastatin, pravastatin, fluvastatin, simvastatin, atorvastatin, rosuvastatin, and pitavastatin. In some embodiments, the solid oral composition comprises 1-MNA or a pharmaceutically acceptable salt thereof and a fibric acid derivative (e.g., gemfibrozil, fenofibrate, and fenofibric acid). In some embodiments, the solid oral composition comprises 1-MNA or a pharmaceutically acceptable salt thereof and a bile acid sequestrate (e.g., cholestyramine, colestipol, and colesevelam). In some embodiments, the solid oral composition comprises 1-MNA or a pharmaceutically acceptable salt thereof and cholesterol absorption inhibitors (e.g., ezetimibe). In some embodiments, the solid oral composition comprises 1-MNA or a pharmaceutically acceptable salt thereof, aspirin and a statin.

In some embodiments, the pharmaceutical composition comprising 1-MNA or a pharmaceutically acceptable salt thereof and the one or more additional agents is an oral pharmaceutical composition. In some embodiments, the oral pharmaceutical composition is a solid oral composition. In some embodiments, the solid oral pharmaceutical composition is a tablet, a capsule, a gelcap, a caplet, or a lozenge.

In compositions (e.g., solid oral compositions) comprising 1-MNA or a pharmaceutically acceptable salt thereof and one or more additional active agents, the amount of the 1-MNA or a pharmaceutically acceptable salt thereof and the one or more additional agents can be determined in view of the daily dose of each drug contained in the composition, dosing frequency, and manufacturing considerations, which can be determined by those of ordinary skill in the art. In some embodiments, the amount of the 1-MNA or a pharmaceutically acceptable salt thereof is as disclosed above. Methods and excipients for making oral pharmaceutical compositions are disclosed above.

### Tests for Determining Blood or Serum CRP Levels

There are generally two types of tests for measuring blood or serum CRP levels. The first type of test is less sensitive (e.g., ELISA) and is useful for monitoring general inflammatory changes in patients with inflammatory diseases (e.g., infection and rheumatoid arthritis) where the CRP levels are higher than 10 mg/L. CRP tests are available from commercial diagnostic labs, e.g., the standard or conventional CRP tests available from Quest Diagnostics.

The second type of test is a high sensitive CRP or hs-CRP tests that measures low levels of CRP with a detection sensitivity of, e.g., 0.2 mg/L or 0.04 mg/L. Blood or serum CRP levels less than 10 mg/L are typically tested using hs-CRP tests, which are used to establish and monitor cardiovascular risk.

hs-CRP tests are available from commercial diagnostic labs. Exemplary hs-CRP tests include Cardio IQ^{®} hs-CRP or hs-CRP tests provided by Quest Diagnostics. As CRP levels increase with acute infection and trauma, testing should be avoided e.g., within a 2-to 3-week window in patients who have had an infection or other acute illness. Individuals with clinically apparent inflammatory conditions such as rheumatoid arthritis or lupus have elevated CRP levels. hs-CRP evaluation for the purpose of cardiovascular risk evaluation in such patients should be avoided until at least 2 weeks after the inflammatory responses has resolved.

Blood and serum CRP tests can be carried out with and without fasting or during different time of the day as CRP levels do not fluctuate significantly at different times or under the different conditions.

### Example

### Treatment with 1-MNA Chloride Reduces Blood or Serum CRP Level

### Study design

The study was a randomized, double-blind, placebo controlled, forced dose-escalation, multicenter study.

### Inclusion Criteria

- Patients were at least 18 years of age and ≤ 80 years of age at the time of informed consent;
- Women of childbearing potential must have a negative urine pregnancy test at screening and visit 4. Women were considered not of childbearing potential if they:
   a. had a hysterectomy or tubal ligation prior to visit 1;
   b. were postmenopausal defined as no menses for 12 months or a FSH level in the menopausal range;
      Women of childbearing potential must agree to use an effective method of birth control throughout the study. Acceptable means of birth control include: implantable contraceptives, injectable contraceptives, oral contraceptives, transdermal contraceptives, intrauterine devices, male or female condoms with spermicide, abstinence, or a sterile sexual partner;
- Patients who at visits 2 and 3 (Weeks -4 and -2) demonstrated mean LDL-cholesterol at the levels at which lipid-modifying drug therapy is not indicated according to investigator judgment under ATP III guidelines (available at http://www.nhlbi.nih.gov/guidelines/cholesterol/atp3full.pdf);
- Patients who demonstrated mean serum triglycerides ≥ 200 mg/dL (2.26 mmol/L) but ≤ 500 mg/dL (5.65 mmol/L) as measured at 2 sequential visits during the dietary controlled baseline period (Visits 2 and 3 or Visits 3 and 3a) and having lower level within 25% of upper level (higher value minus lower value)/higher value < 0.25);
- Patients who were willing to maintain a stable diet and physical activity level throughout the study;
- Patients who were willing and able to sign the information and consent form and follow the protocol including availability for all visits/telephone follow-up for approximately 26 weeks.

### Exclusion Criteria

- Patients who were pregnant, planning to become pregnant during the study, or nursing
- Patients who had with clinically significant electrocardiographic abnormalities at visit 1 or visit 4;
- Patients who had body mass index > 45 kg/m2 at visit 1;
- Patients who had weight change of > 5% of initial body weight between visits 1 and 4 (randomization);
- Patients who had poorly controlled diabetes defined as a hemoglobin A1c > 9.5% prior to visit 4 (randomization);
- Patients who had evidence of hepatic disease (ALT or AST greater than 2.0 ULN, bilirubin greater than 1.5 ULN, or cirrhosis) at visit 1;
- Patients who had renal dysfunction defined as glomerular filtration rate (GFR) < 60 mL/min/1.73 m2 at visit 1;
- Patients who had hypothyroidism that was not treated or not stable for at least 6 months prior to study entry;
- Patients who had poorly controlled hypertension defined as a mean systolic blood pressure above 160 mm Hg and/or diastolic blood pressure above 100 mmHg at visit 1. In patients who had end-organ damage, mean systolic blood pressure above 140 mm Hg and mean diastolic blood pressure above 90 mm Hg at visit 1;
- Patients who had severe hypotension defined as systolic blood pressure ≤ 90 mm Hg or diastolic blood pressure ≤ 60 mm Hg AND symptomatic;
- Patients who had an active peptic ulcer;
- Patients who had known intolerance (except flush) or allergic to placebo or investigational product;
- Patients who had any known history of coronary artery disease, cerebrovascular disease or peripheral arterial disease;
- Patients who used any of the following lipid modifying medications/supplements from the time after screening to the conclusion of the study:
   Niacin (nicotinic acid) or niacinamide (nicotinamide)
   Fibrates or fibric acid derivatives including fenofibrate, gemfibrozil, clofibrate
   Bile acid sequestrants including cholestyramine, colesevelam, colestipol hydrochloride
   HMG-CoA reductase inhibitors (statins) including atorvastatin, cerivastatin, fluvastatin, lovastatin, pravastatin, simvastatin, rosuvastatin Ezetimibe
   Omega-3 fatty acids
   Supplements containing flaxseed, tryptophan, fish oil, or algal oil.
   Sterol/stanol products
   Red yeast rice supplements or soy isoflavone supplements
   Dietary fiber supplements including > 2 teaspoonfuls of Metamucil^{®} or psyllium containing supplements per day.
   Other natural health products or prescription agents judged by the investigator to have the potential to alter serum lipid levels in an individual subject.
- Patients who had a history of angina or myocardial infarction;
- Patients who based on the investigator's judgment had clinically significant hyperuricemia or with a history of gouty arthritis;
- Patients who had known nephritic syndrome or history of >3 g protein/day in urine;
- Patients who had known familial lipoprotein lipase deficiency, apo CII deficiency, or familial dysbetalipoproteinemia;
- Patients who required peritoneal dialysis or hemodialysis for renal insufficiency;
- Patients who had a history of malignancy, except patients who had been disease-free for > 5 yrs, or resected basal or squamous cell skin carcinoma or cervical carcinoma in situ;
- Patients who had a history of bariatric surgery;
- Patients who had a history of pancreatitis, except secondary to cholelithiasis;
- Patients who anticipated major surgery during the study;
- Patients who had treatments with weight loss drugs or programs during the trial;
- Patients who had treatments with HIV-protease inhibitors, cyclophosphamide or isotretinoin;
- Patients who had treatments with tamoxifen, estrogens, or progestins that has not been stable for > 4 weeks prior to screening at visit 1;
- Patients who had routine or anticipated use of all systemic corticosteroids at visit 1, Local, topical, inhaled, or nasal corticosteroids were permitted;
- Patients who had blood donation of > pint (0.5 L) within 30 days prior to screening, or had plasma donation within 7 days prior to screening at visit 1;
- Patients who had consumption of > 14 alcoholic drinks per week (1 drink = 12 oz beer, 5 oz wine, or 1.5 oz hard liquor) at visit 1;
- Patients who had a history of drug abuse;
- Patients who participated in another clinical trial within 30 days of signing the information and consent form;
- Patients who did not comply single blind investigational product (< 80% investigational product) or diet as per local judgment between visits 1 and 4;
- Patients who had any condition or therapy that the investigator believed might pose a risk to the patient or make participation in the study not in the patient's best interest;
- Patients who had poor mental function or any other reason to expect patient difficulty in complying with the requirements of the study.

Following signature of informed consent, 164 patients received 1000 mg placebo three times daily with meals in a single-blind manner for a dietary-controlled baseline period of 6 to 8 weeks. From the sample of eligible subjects who completed the 6 to 8 week dietary-controlled baseline period, 71 subjects (22 received placebo and 49 received 1-MNA chloride) meeting all inclusion and no exclusion criteria were randomized in a double-blind manner (3:1 ratio) to the treatment arm. The treatment periods were: weeks 1 and 2 two 500 mg tablets administered three times daily with meals (total daily dose 3000 mg); weeks 3 to 14 two 1000 mg tablets administered three times daily with meals (total daily dose 6000 mg). Down titration to 3000 mg daily was allowed in the event that a subject could not tolerate the 6000 mg daily treatment for the stipulated period. Under this scenario, the down-titrated subject remained on the tolerated dose for the duration of the trial. hs-CRP blood levels were evaluated during the baseline period, upon randomization and throughout the active treatment period. All blood samples were collected following a 12-hour fast. Throughout the study, the subjects were required to adhere to a heart-healthy diet and abstain from/minimize ethyl alcohol intake. Safety and tolerability were assessed throughout the trial through the evaluation of physical exams, electrocardiograms, routine hematology ad blood chemistry testing, vital signs and adverse events.

The hs-CRP results of the study are summarized in Table 1.

**Table 1. Summary of and statistical analysis for change in C-reactive protein**

| **C-reactive protein (mg/L)** | | **Placebo** | **1-MNA Chloride** |
|---|---|---|---|
| Baseline | n | 22 | 49 |
| | Mean ± SD | 5.5 ± 5.5 | 4.0 ± 5.4 |
| | Geometric mean | 4.0 | 2.9 |
| End of study | n | 20 | 45 |
| | Mean ± SD | 5.3 ± 4.5 | 3.3 ± 3.2 |
| | Geometric mean | 3.7 | 2.4 |
| Change from baseline to end of study | n | 20 | 45 |
| | Mean ± SD | -0.5 ± 2.1 | -0.8 ± 4.7 |
| | Adjusted geometric mean percent change from baseline | 1.21 | -15.66 |

The results show that administration of 1-MNA chloride lowers blood or serum CRP level in a subject by about 16% from baseline.

Statistical analysis for change from baseline to end of study in CRP is shown in Table 2 below.

**Table 2. Statistical Analysis of CRP Change from Baseline to End of Study**

| Treatment | Adjusted geometric mean % change (95% CI) | p-value | Placebo-adjusted geometric mean % change (95% CI) | p-value |
|---|---|---|---|---|
| Placebo | 1.21 (-13.95, 19.05) | 0.8779 | -16.67 (-33.59, 4.57) | 0.1130 |
| 1-MNA chloride | -15.66 (-28.40, -0.66) | 0.0419 | | |

In the analysis, adjusted geometric mean percent change was obtained by exponentiating the adjusted mean from the ANCOVA model, then subtracting 1 and multiplying by 100. Bounds of the 95% confidence intervals were obtained similarly. Based on the ANCOVA model of the change in log-transformed CRP including treatment group and log-transformed baseline value, the change in CRP level in blood in this study was approximately -17% relative to placebo.

The present invention is defined by the following claims

## Claims

1. A pharmaceutically-acceptable salt of 1-methylnicotinamide (1-MNA) for use in the prevention of a cardiovascular disease in a human subject having a high risk of development of a cardiovascular disease,
wherein a pharmaceutically acceptable salt of 1-methylnicotinamide (1-MNA) is used in a therapeutically effective amount, and,
wherein the cardiovascular disease is selected from the group consisting of coronary heart disease (CHD), peripheral artery disease, carotid artery disease, congestive heart failure, stroke, myocardial infarct, angina or a combination thereof,
and wherein prior to administering the human subject has a blood or serum C-reactive protein (CRP) level of less than 10 mg/L,
and wherein the human subject is a non-dyslipidemic human subject,
and wherein a non-dyslipidemic human subject is a human subject having a normal total blood cholesterol level (≤200 mg/dL), a normal LDL-cholesterol level (≤130 mg/dL), a normal TG level (≤150 mg/dL), or a normal HDL-cholesterol level (≥60 mg/dL), or a combination thereof,
and wherein the high risk occurs when the human subject has CRP level between 3 mg/L and 10 mg/L.

2. A pharmaceutically-acceptable salt of 1-methylnicotinamide (1-MNA) for use according to claim 1, wherein the human subject has a blood C-reactive protein (CRP) in a range selected from the group consisting of: less than 1.0 mg/L, less than 0.5 mg/L, between 0.5 mg/L to less than 1.0 mg/L, between 1.0 mg/L to less than 10.0 mg/L, more preferably between 0.5 mg/L to less than 1.0 mg/L, preferably less than 1.0 mg/L, between 1.0 mg/L to less than 3.0 mg/L, between 1.0 mg/L to 2.0 mg/L, between 1.0 mg/L to 2.7 mg/L, or between 2.0 to less than 3.0 mg/L, 3.0 mg/L to 5.0 mg/L, between 3.0 mg/L to 7.0 mg/L, or between 3.0 mg/to less than 10.0 mg/L.

3. A pharmaceutically-acceptable salt of 1-methylnicotinamide (1-MNA) for use according to claim 2, wherein the human subject has a blood C-reactive protein (CRP) in a range of between 1.0 mg/L to less than 10 mg/L.

4. A pharmaceutically-acceptable salt of 1-methylnicotinamide (1-MNA) for use according to any of claims 1-3, wherein the therapeutically effective amount of a pharmaceutically acceptable salt of 1-MNA is for oral administration.

5. A pharmaceutically-acceptable salt of 1-methylnicotinamide (1-MNA) for use according to any of claims 1-4, wherein the pharmaceutically acceptable salt is 1-methylnicotinamide chloride.

6. A pharmaceutically-acceptable salt of 1-methylnicotinamide (1-MNA) for use according to any of claims 1-5, wherein the therapeutically effective amount of a pharmaceutically acceptable salt of 1-MNA is in the range of from 1000 to 8000 mg, from 1000 mg to 7000 mg, from 1000 mg to 6000 mg, from 1000 mg to 5000 mg, from 1000 mg to 4000 mg, from 1000 mg to 3000 mg, from 1000 mg to 2000 mg, from 2000 mg to 8000 mg, from 2000 mg to 7000 mg, from 2000 mg to 6000 mg, from 2000 mg to 5000 mg, from 2000 mg to 4000 mg, from 2000 mg to 3000 mg, from 3000 mg to 8000 mg, from 3000 mg to 7000 mg, from 3000 mg to 6000 mg, from 3000 mg to 5000 mg, from 3000 mg to 4000 mg, from 4000 mg to 8000 mg, from 4000 mg to 7000 mg, from 4000 mg to 6000 mg, from 4000 to 5000 mg, from 5000 mg to 8000 mg, from 5000 mg to 7000 mg, from 5000 mg to 6000 mg, from 6000 mg to 8000 mg, from 6000 mg to 7000 mg, from or 7000 mg to 8000 mg, per day;
preferably 1000 mg, 2000 mg, 3000 mg, 4000, 5000 mg, 6000 mg, 7000 mg, or 8000 mg, per day, even more preferably 1000 mg, 3000 mg, or 6000 mg, per day.

7. A pharmaceutically-acceptable salt of 1-methylnicotinamide (1-MNA) for use according to any of claims 1-6, wherein the therapeutically effective amount of a pharmaceutically acceptable salt 1-MNA is for once a day, twice a day, or three times a day administration.

## Patentansprüche

1. Ein pharmazeutisch zulässiges Salz von 1-Methylnicotinamid (1-MNA) zur Verwendung für Vorbeugung von kardiovaskulärer Krankheit bei einem menschlichen Probanden mit einem hohen Risiko für die Entwicklung kardiovaskulärer Krankheit,
wobei das pharmazeutisch zulässige Salz von 1-Methylnicotinamid (1-MNA) in einer therapeutisch wirksamen Dosis verabreicht wird, und
wobei die kardiovaskuläre Krankheit aus einer Gruppe ausgewählt wird, die aus koronarer Herzkrankheit (KHK), peripherer Arterien-, Halsschlagadererkrankung, kongestiver Herzinsuffizienz, Schlaganfall, Myokardinfarkt, Angina oder einer Kombination von diesen Erkrankungen besteht,
und wobei der menschliche Proband vor der Verabreichung einen Wert von C-reaktivem Protein (CRP) im Blut oder Serum von weniger als 10 mg/L aufweist,
und wobei der menschliche Proband ein menschlicher Proband ist, der nicht an Dyslipidämie leidet,
und wobei dieser nicht an Dyslipidämie leidende Proband einen normalen Gesamtcholesterinspiegel (≤200 mg/dL), einen normalen LDL-Cholesterinspiegel (≤130 mg/dL), einen gesunden TG-Spiegel (≤150 mg/dL) oder einen normalen HDL-Cholesterinspiegel (≥60 mg/dL) oder eine Kombination davon aufweist,
und wobei ein hohes Risiko auftritt, wenn der menschliche Proband einen CRP-Wert zwischen 3 mg/L und 10 mg/L aufweist.

2. Pharmazeutisch zulässiges Salz von 1-Methylnicotinamid (1-MNA) zur Verwendung gemäß Anspruch 1, wobei der menschliche Proband von C-reaktivem Protein (CRP) im Blut in einem Bereich aufweist, der ausgewählt ist aus der folgenden Gruppe der Werte: weniger als 1,0 mg/L, weniger als 0,5 mg/L, zwischen 0,5 mg/L und weniger als 1,0 mg/L, zwischen 1,0 mg/L und weniger als 10.0 mg/L, weiter bevorzugt zwischen 0,5 mg/L und weniger als 1,0 mg/L, vorzugsweise weniger als 1,0 mg/L, zwischen 1,0 mg/L und weniger als 3,0 mg/L, zwischen 1,0 mg/L und 2,0 mg/L, zwischen 1,0 mg/L und 2,7 mg/L oder zwischen 2,0 und weniger als 3,0 mg/L, 3,0 mg/L und 5,0 mg/L, zwischen 3,0 mg/L und 7,0 mg/L oder zwischen 3,0 mg/L und weniger als 10,0 mg/L.

3. Ein pharmazeutisch zulässiges Salz von 1-Methylnicotinamid (1-MNA) zur Verwendung gemäß Anspruch 2, wobei der menschliche Proband von C-reaktivem Protein (CRP) im Blut in einem Bereich zwischen 1,0 mg/L und weniger als 10 mg/L aufweist.

4. Ein pharmazeutisch zulässiges Salz von 1-Methylnicotinamid (1-MNA) zur Verwendung nach einem der Ansprüche 1 - 3, wobei die therapeutisch wirksame Dosis des pharmazeutisch zulässigen Salzes von 1-MNA zur oralen Verabreichung bestimmt ist.

5. Ein pharmazeutisch zulässiges Salz von 1-Methylnicotinamid (1-MNA) zur Verwendung nach einem der Ansprüche 1 - 4, wobei das pharmazeutisch zulässiges Salz 1-Methylnicotinamidchlorid ist.

6. Ein pharmazeutisch zulässiges Salz von 1-Methylnicotinamid (1-MNA) zur Verwendung nach einem der Ansprüche 1 - 5, wobei die therapeutisch wirksame Dosis des pharmazeutisch zulässigen Salzes von 1-MNA im Bereich von 1000 bis 8000 mg, von 1000 mg bis 7000 mg, von 1000 mg bis 6000 mg, von 1000 mg bis 5000 mg, von 1000 mg bis 4000 mg, von 1000 mg bis 3000 mg, von 1000 mg bis 2000 mg, von 2000 mg bis 8000 mg, von 2000 mg bis 7000 mg, von 2000 mg bis 6000 mg, von 2000 mg bis 5000 mg, von 2000 mg bis 4000 mg, von 2000 mg bis 3000 mg, von 3000 mg bis 8000 mg, von 3000 mg bis 7000 mg, von 3000 mg bis 6000 mg, von 3000 mg bis 5000 mg, von 3000 mg bis 4000 mg, von 4000 mg bis 8000 mg, von 4000 mg bis 7000 mg, von 4000 mg bis 6000 mg, von 4000 bis 5000 mg, von 5000 mg bis 8000 mg, von 5000 mg bis 7000 mg, von 5000 mg bis 6000 mg, von 6000 mg bis 8000 mg, von 6000 mg bis 7000 mg, von 7000 mg bis 8000 mg pro Tag;
vorzugsweise 1000 mg, 2000 mg, 3000 mg, 4000 mg, 5000 mg, 6000 mg, 7000 mg oder 8000 mg pro Tag,
noch bevorzugter 1000 mg, 3000 mg oder 6000 mg pro Tag liegt.

7. Ein pharmazeutisch zulässiges Salz von 1-Methylnicotinamid (1-MNA) zur Verwendung nach einem der Ansprüche 1 - 6, wobei die therapeutisch wirksame Dosis des pharmazeutisch zulässigen Salzes 1-MNA ist für eine einmalige, zweimalige oder dreimalige Verabreichungsdosis pro Tag.

## Revendications

1. Sel pharmaceutiquement acceptable de 1-méthylnicotinamide (1-MNA) pour utilisation dans la prévention d'une maladie cardiovasculaire chez un sujet humain présentant un risque élevé de développement d'une maladie cardiovasculaire, dans lequel un sel pharmaceutiquement acceptable de 1-méthylnicotinamide (1-MNA) est utilisé dans une quantité thérapeutiquement efficace, et,
où la maladie cardiovasculaire est choisie dans le groupe constitué par la maladie coronarienne, la maladie artérielle périphérique, la maladie de l'artère carotide, l'insuffisance cardiaque congestive, l'accident vasculaire cérébral, l'infarctus du myocarde, l'angine de poitrine ou une combinaison de ces maladies,
et dans lequel, avant l'administration, le sujet humain présente un taux sanguin ou sérique de protéine C-réactive (CRP) inférieur à 10 mg/L,
et dans lequel le sujet humain est un sujet humain non dyslipidémique,
et dans lequel un sujet humain non dyslipidémique est un sujet humain ayant un taux de cholestérol sanguin total normal (≤200 mg/dL), un taux de cholestérol LDL normal (≤130 mg/dL), un taux de TG normal (≤150 mg/dL), ou un taux de cholestérol HDL normal (≥60 mg/dL), ou une combinaison de ceux-ci,
et dans lequel le risque est élevé lorsque le sujet humain présente un taux de CRP compris entre 3 mg/L et 10 mg/L.

2. Sel pharmaceutiquement acceptable de 1-méthylnicotinamide (1-MNA) pour l'utilisation selon la revendication 1, dans lequel le sujet humain a une protéine C-réactive (CRP) sanguine dans une gamme choisie dans le groupe consistant en : moins de 1,0 mg/L, moins de 0,5 mg/L, entre 0,5 mg/L et moins de 1,0 mg/L, entre 1,0 mg/L et moins de 10. 0 mg/L, de préférence entre 0,5 mg/L et moins de 1,0 mg/L, de préférence moins de 1,0 mg/L, entre 1,0 mg/L et moins de 3,0 mg/L, entre 1,0 mg/L et 2,0 mg/L, entre 1,0 mg/L et 2,7 mg/L, ou entre 2,0 et moins de 3,0 mg/L, entre 3,0 mg/L et 5,0 mg/L, entre 3,0 mg/L et 7,0 mg/L, ou entre 3,0 mg/à moins de 10,0 mg/L.

3. Sel pharmaceutiquement acceptable de 1-méthylnicotinamide (1-MNA) pour l'utilisation selon la revendication 2, dans lequel le sujet humain a une protéine C-réactive (CRP) sanguine comprise entre 1,0 mg/L et moins de 10 mg/L.

4. Sel pharmaceutiquement acceptable de 1-méthylnicotinamide (1-MNA) à utiliser selon l'une des revendications 1 à 3, dans lequel la quantité thérapeutiquement efficace d'un sel pharmaceutiquement acceptable de 1-MNA est destinée à l'administration orale.

5. Sel pharmaceutiquement acceptable de 1-méthylnicotinamide (1-MNA) pour l'utilisation selon l'une des revendications 1 - 4, dans lequel le sel pharmaceutiquement acceptable est le chlorure de 1-méthylnicotinamide.

6. Sel pharmaceutiquement acceptable de 1-méthylnicotinamide (1-MNA) à utiliser selon l'une des revendications 1 - 5, dans lequel la quantité thérapeutiquement efficace d'un sel pharmaceutiquement acceptable de 1-MNA est comprise entre 1 000 et 8 000 mg, entre 1 000 et 7 000 mg, entre 1 000 et 6 000 mg, entre 1 000 et 5 000 mg, entre 1 000 et 4 000 mg, entre 1 000 et 3 000 mg, entre 1 000 et 2 000 mg, entre 2 000 et 8 000 mg, entre 2 000 et 7 000 mg, entre 2 000 et 6 000 mg, entre 2 000 et 5 000 mg, entre 2 000 et 4 000 mg, entre 2 000 et 3 000 mg, entre 2 000 et 3 000 mg, entre 2 000 et 6 000 mg, entre 2 000 et 3 000 mg, entre 3 000 et 8 000 mg, entre 3 000 et 7 000 mg, entre 3 000 et 6 000 mg, entre 3 000 et 5 000 mg, entre 3 000 et 7 000 mg, entre 3 000 et 6 000 mg, entre 3 000 et 5 000 mg, entre 1 000 et 3 000 mg, entre 1 000 et 3 000 mg, de 2 000 mg à 5 000 mg, de 2 000 mg à 4 000 mg, de 2 000 mg à 3 000 mg, de 3 000 mg à 8 000 mg, de 3 000 mg à 7 000 mg, de 3 000 mg à 6 000 mg, de 3 000 mg à 5 000 mg, de 3 000 mg à 4 000 mg, de 4 000 mg à 8 000 mg, de 4 000 mg à 7 000 mg, de 4 000 mg à 6 000 mg, de 4 000 à 5 000 mg, de 5 000 mg à 8 000 mg, de 5 000 mg à 7 000 mg, de 5 000 mg à 6 000 mg, de 6 000 mg à 8 000 mg, de 6 000 mg à 7 000 mg, de 7 000 mg à 8 000 mg, par jour ;
de préférence 1 000 mg, 2 000 mg, 3 000 mg, 4 000 mg, 5 000 mg, 6 000 mg, 7 000 mg ou 8 000 mg par jour, de préférence encore 1 000 mg, 3 000 mg ou 6 000 mg par jour.

7. Sel pharmaceutiquement acceptable de 1-méthylnicotinamide (1-MNA) pour l'utilisation selon l'une des revendications 1 - 6, dans laquelle la quantité thérapeutiquement efficace d'un sel pharmaceutiquement acceptable de 1-MNA est pour une administration une fois par jour, deux fois par jour, ou trois fois par jour.
